# EUROPEAN PATENT APPLICATION

(11) **EP 2 070 537 A1**
(43) Date of publication of application: **17.06.2009**
(21) Application number: 08170429.8
(22) Date of filing: 02.12.2008
(51) Int. Cl.: A61K 31/4745, C07D 491/22, A61P 35/00

(54) **Crystalline polymorphs of topotecan hydrochloride and methods for the preparation thereof**

(30) Priority: 04.12.2007 IT MI20072268
(71) Applicant: Antibioticos S.p.A., 20090 Rodano (MI) (IT)
(72) Inventor: Pozzi, Giovanni, 20045 BESANA BRIANZA (MI) (IT); Ghetti, Paolo, 20090 Rodano (MI) (IT); Balsamo, Gaetano, 20096, PIOLTELLO (MI) (IT); Negri, Ettore, 20024, GARBAGNATE MILANESE (MI) (IT); Mazzoni, Andrea, 20090 Rodano (MI) (IT); Alpegiani, Marco, 20132, MILANO (IT); Bedeschi, Angelo, 20024 GARBAGNATE MILANESE (MI) (IT); Pizzocaro, Roberta, 20024 GARBAGNATE MILANESE (MI) (IT)
(74) Representative: Minoja, Fabrizio

(57) **Abstract**

The invention relates to two novel crystalline form of Topotecan hydrochloride (**Ia**)

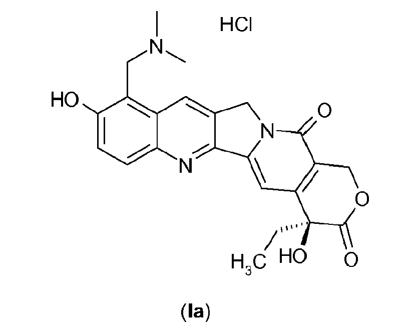

Herein referred to as forms α and β, characterized by high purity and whose preparation is advantageous from the industrial point of view. Form α can be in fact conveniently prepared starting from 10-hydroxy-camptothecin, whereas form β can be prepared starting from form α.

## Description

### Field of the invention

The present invention relates to antitumor medicaments, in particular to Topotecan **(I)**

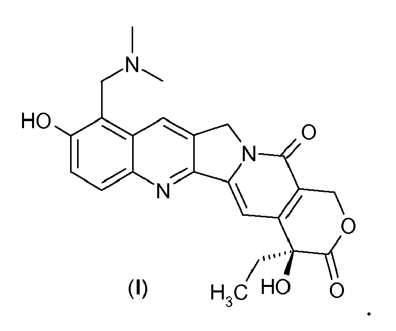

### Technological background

Topotecan **(I)** [(4S)-10-[(dimethylamino)methyl]-4-ethyl-4,9-dihydroxy-1H-pirano[3',4':6,7] indolizino-[1,2-b]quinoline-3,14(4H,12H)-dione] is an antineoplastic medicament that inhibits topoisomerase I. It is synthesized from camptothecin as described for example in US 5,004,758, US 2007/0149783, US 5,734,056, US 6,660,861 and is commonly used in therapy in the form of the hydrochloride salt.

Polymorphic forms of active principles and of raw materials for pharmaceutical use are particularly interesting, because they affect, for example, the stability of the powders and of the finished pharmaceutical forms, the processes for their preparation and the bioavailability of the active ingredient.

Three different polymorphic forms of Topotecan hydrochloride are known:
- Topotecan monohydrochloride pentahydrate with water content ranging from 10.5 to 16.5%, disclosed in WO N° 2005/046608, US N° 2007/117832 and Journal of Pharmaceutical and Biomedical Analysis 40 (2006) 1080 - 1088 "A study of variable hydration states in Topotecan hydrochloride";
- Topotecan hydrochloride forms I and II, disclosed in US N° 2007/0149783;
- Topotecan hydrochloride form A with water content ranging from 10 to 12% (WO 2007/042799, US 2007/105885).

### Disclosure of the invention

The invention relates to two polymorphic forms of Topotecan hydrochloride **(Ia)** 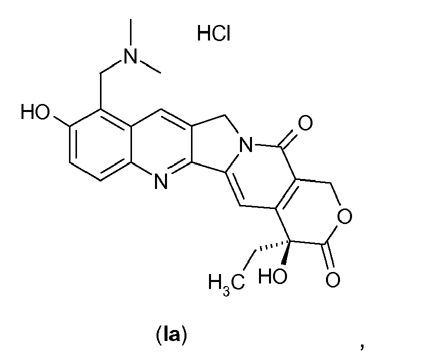 in the following referred to as form α and β. Due to their high crystallinity, these polymorphs are chemically stable, highly pure and very easily filterable, so they can be easily recovered (form α) or purified from reaction mixtures (form β). For these reasons, polymorphs α and β can be produced on an industrial scale in a convenient and highly productive way.

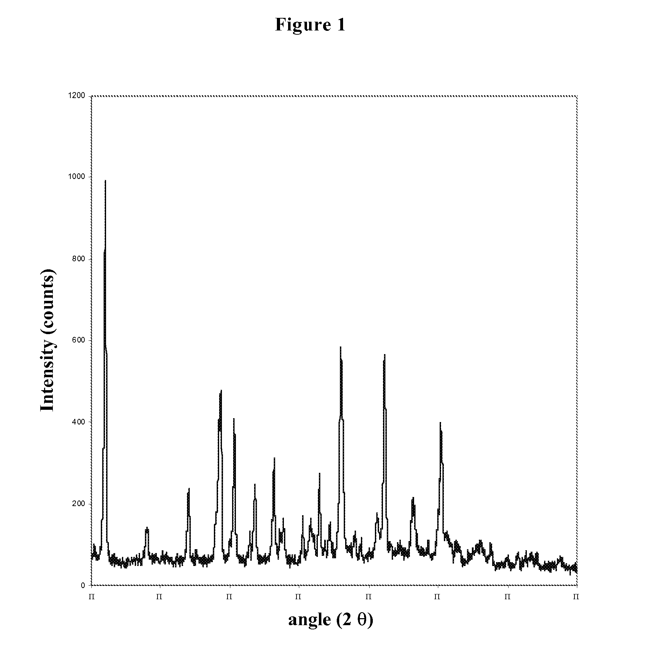

Form α is obtained by reaction of 10-hydroxy-camptothecin **(II)** 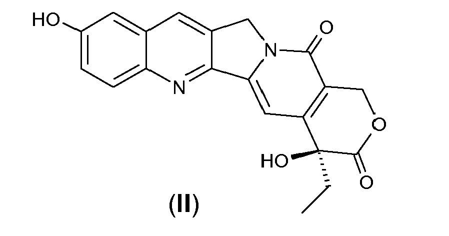 with a large excess of aqueous formaldehyde and aqueous dimethylamine in acetic acid and in a straight or branched C₂-C₄ alcohol, preferably ethanol or isopropanol, subsequent treatment with a controlled excess of hydrochloric acid, concentration of the reaction mixture and subsequent crystallization by addition of an alcohol as defined above, preferably aqueous isopropanol or isopropanol. Form α has an XRPD spectrum (powder X-ray) with the characteristic peaks listed in the table 1 and graphically illustrated in figure 1.

**Table 1**

| **Angle (2-Theta)** | **Value d (Angstrom)** | **Intensity (%)** |
|---|---|---|
| 6.1 | 14.55 | 100.0 |
| 12.0 | 7.34 | 24.2 |
| 14.3 | 6.17 | 48.1 |
| 15.3 | 5.77 | 40.9 |
| 16.8 | 5.27 | 24.1 |
| 18.2 | 4.87 | 31.6 |
| 21.5 | 4.13 | 25.6 |
| 23.0 | 3.86 | 59.1 |

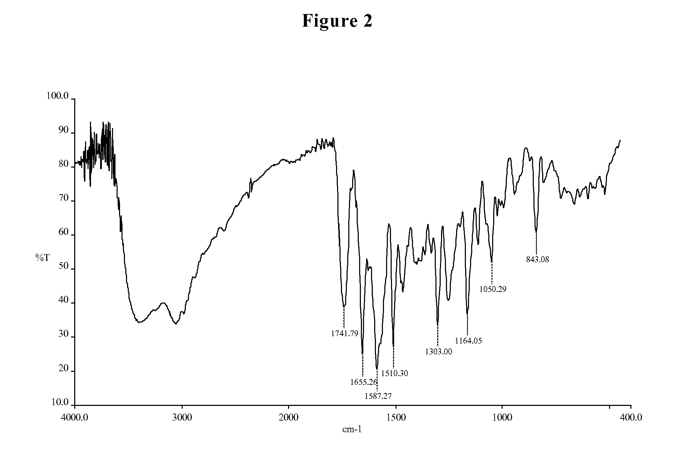

The IR spectrum of the α form is reported in figure 2.

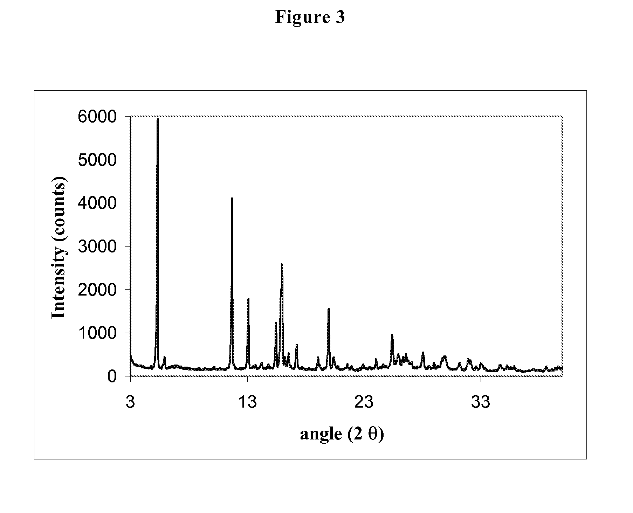

Crystalline form β is obtained by treatment of form α in aqueous isopropanol and has an XRDP spectrum characterized by the peaks reported in the following table 2 and graphically illustrated in figure 3.

**Table 2**

| **Angle (2-Theta)** | **Value d (Angstrom)** | **Intensity (%)** |
|---|---|---|
| 5.3 | 16.59 | 100.0 |
| 11.7 | 7.56 | 21.7 |
| 13.1 | 6.76 | 11.6 |
| 15.5 | 5.73 | 8.8 |
| 16.0 | 5.55 | 38.4 |
| 16.6 | 5.35 | 5.4 |
| 17.2 | 5.14 | 6.0 |
| 20.0 | 4.44 | 13.9 |
| 25.4 | 3.50 | 10.4 |

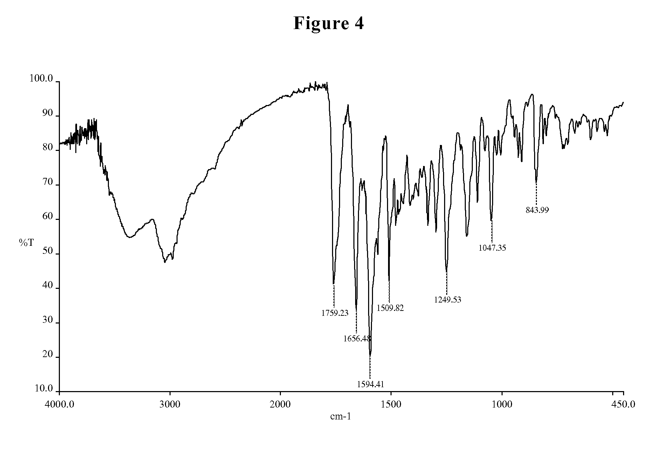

The IR spectrum of form β is reported in figure 4.

In more detail, the preparation of form α is carried out reacting 10-hydroxy camptothecin with 10 - 13 equivalents of aqueous dimethylamine per equivalent of 10-hydroxy camptothecin and 4 - 18 equivalents of aqueous formaldehyde per equivalent of 10-hydroxy camptothecin in glacial acetic acid and alcohol as defined above to give a solution of Topotecan. Dimethylamine can be used as gas or as water solution or dissolved in an organic solvent. According to a preferred embodiment of the invention, dimethylamine and formaldehyde are used, both as aqueous solutions with a concentration ranging from 35 to 45% w/w, more preferably of 40% w/w. Preferably, glacial acetic acid is used in amount equal to 8 - 68 volumes with respect to 10-hydroxy camptothecin.

Preferably, the alcohol used in the solution of 10-hydroxy camptothecin is ethanol or isopropanol in a volumetric ratio of 3 - 28 with respect to 10-hydroxy camptothecin.

The synthesis can be carried out at temperatures ranging from about 20°C to about 60°C, for a time sufficient to complete the reaction, from 1 to 24 hours or for a longer time.

After completion of the reaction, the mixture is added with 1 - 2 equivalents of concentrated hydrochloric acid, in the gaseous form or as a solution in water or in an organic solvent, per equivalent of dimethylamine. Preferably, concentrated hydrochloric acid as aqueous solution at a concentration ranging from 35 to 37% w/w, preferably of 37% w/w, is used. The treatment with the acid can be carried out at temperatures from about 10°C to about 60°C.

After treatment with the acid, the reaction mixture is concentrated under reduced pressure to a final volume of 8 - 10 volumes with respect to 10-hydroxy camptothecin. Concentration is carried out a temperature from about 10°C to about 60°C and residual pressure lower than 50 mbar.

Crystallization of polymorph α of Topotecan hydrochloride is accomplished by addition of isopropanol or aqueous isopropanol at temperatures ranging from 45°C to 52°C in a time interval ranging from 60 to 120 min, preferably of 90 minutes. Preferably, aqueous isopropanol containing 5% water is used. When addition of the alcohol is complete, the crystallized product is cooled to a temperature ranging from 15°C to 25°C, preferably to 20°C and the solid is recovered by filtration with one of the following techniques: gravity, pressure, suction, centrifugation, decantation.

Topotecan hydrochloride crystalline form α obtained with the above process has an HPLC purity of 99.5% or higher.

Topotecan hydrochloride crystalline form α can be dried in an oven under vacuum, in a static dryer or in other dryer apparatuses. Drying can be effected at pressure lower than 50 mbar at a temperature from 20°C to 30°C for a sufficient time for reaching the desired purifty level, usually from 1 to 20 hours.

Topotecan hydrochloride crystalline form β can be obtained suspending form α in aqueous isopropanol at temperatures ranging from 48°C to 52°C, preferably of 50°C for at least 60 minutes. Afterwards, this suspension is cooled to a temperature ranging from 15°C to 25°C, preferably to 20°C and the solid is recovered by filtration with one of the following techniques: gravity, pressure, suction, centrifugation, decantation.

Topotecan hydrochloride crystalline form β obtained with the disclosed process has an HPLC purity of 99.7% or higher.

Topotecan hydrochloride crystalline form β can be dried in vacuum dryers, static dryers or other drying apparatuses.

Drying can be carried out under pressure lower than 50 mbar, at a temperature from 20°C to 30°C for a sufficient time for reaching the desired purity, usually for 1 to 20 hours. After drying, Topotecan hydrochloride crystalline form β can be packaged or optionally hydrated under controlled humidity conditions, usually under 40% to 90% relative humidity and at a temperature from 20 to 30°C until a water content ranging from 3% to 10% is obtained.

A further object of the invention is the use of Topotecan hydrochloride form α and β for the preparation of pharmaceutical compositions. Such pharmaceutical compositions can be prepared according to methods and excipients known to skilled persons, for example according in Remington's "The science and practice of Pharmacy", 21^{st} ed. (Lippincott Williams & Wilkins) and according to the "Handbook of Pharmaceutical Excipients" (Pharmaceutical Press, 5^{th} ed.).

The invention is now illustrated in greater detail by means of some examples.

### EXAMPLES

### Example 1 - Topotecan hydrochloride form α

10-Hydroxy camptothecin (10 g) was dispersed in glacial acetic acid (172 ml), isopropanol (69 ml) and 40% aqueous formaldehyde (28.5 ml), cooling to a temperature of 10°C; thereafter, 40% aqueous dimethylamine was dropped into the suspension at (34.5 ml) in about 40 minutes, without exceeding 13°C. The temperature of the reaction mixture was adjusted at 25°C and stirring was continued until completion of the reaction (HPLC analysis), which required about 23 hours; during this time the reaction mixture turned to a solution. After cooling again to 10°C 37% aqueous hydrochloric acid (34.1 ml) was dropped into the solution in about 30 minutes, without exceeding 12°C. The solvent was distilled off at a pressure lower than 50 mbar a 50°C to obtain a concentrated solution (80 ml), which was heated setting the temperature at 50°C. When the temperature of the concentrated solution had reached 45°C 95% aqueous isopropanol (500 ml) was added drop-by-drop in 90 minutes without exceeding 52°C. The crystallized product was allowed to stand at 50°C for 60 min, then cooled to 20°C and after 60 minutes was filtered with suction. The wet product was washed with 95% aqueous isopropanol (250 ml). After drying under vacuum in a static dryer (residual pressure lower than 50 mbar) 11.3 g Topotecan hydrochloride form α was obtained (99.6%HPLC purity).

### Example 2 - Topotecan hydrochloride form β

Topotecan hydrochloride from example 1 (11.3 g) was suspended in 95% aqueous isopropanol (564 ml) at 50°C for 60 minutes. The suspension was cooled to 20°C and was allowed to stand for 120 minutes, then filtered with suction. The wet product was washed with 95% aqueous isopropanol (282 ml). After drying in a static dryer under vacuum (residual pressure lower than 50 mbar), Topotecan hydrochloride form β (9.7 g) was hydrated under 68% relative humidity. After 18 hours the weight was stable (10.1 g) and Topotecan hydrochloride form β had a 99.7% HPLC purity with a water content of 6.2%.

### Example 3 - Topotecan hydrochloride form α

10-Hydroxy camptothecin (90 g) was dispersed in glacial acetic acid (1548 ml), isopropanol (621 ml) and 40% aqueous formaldehyde (256.5 ml) at a temperature of 10°C, then added drop-by-drop with 40% aqueous dimethylamine (310.5 ml) in about 45 minutes, without exceeding 15°C. The mixture's temperature was adjusted at 25°C and stirring was continued until completion of the reaction (HPLC analysis), which required 22 hours; during this time the reaction mixture turned to a solution. After cooling again to 10°C, 37% aqueous hydrochloric acid was dropped in the solution (307 ml) in about 30 minutes, without exceeding 15°C. The solvent was distilled off at 50°C under pressure lower than 50 mbar, to obtain a concentrated solution (800 ml), which was heated setting the temperature at 50°C. When the temperature reached 45°C, 95% aqueous isopropanol was added drop-by-drop (4500 ml) in 90 minutes, without exceeding 52°C. Crystallization of form β started after addition of the first 1500 ml of alcohol. The crystallized product was allowed to stand 30 minutes at 50°C, cooled to 23°C and filtered with suction after 90 min. The wet product was washed with 95% aqueous isopropanol (ml 2250) and dried under vacuum in a static dryer (residual pressure lower than 50 mbar) to afford 93 g Topotecan hydrochloride form α with 99.6% HPLC purity.

### Example 4 -Topotecan hydrochloride form β

Topotecan hydrochloride form α obtained according to Example 3 (92 g) was treated with 95% aqueous isopropanol (4600 ml) at 50°C for 60 minutes. The suspension was cooled to 20°C and allowed to stand for 120 minutes, then filtered with suction. The wet product was washed with 95% aqueous isopropanol (2300 ml). After drying in a static dryer under vacuum (residual pressure lower than 50 mbar), Topotecan hydrochloride form β (79 g) was hydrated under 56% relative humidity. After 24 hours the weight was stable (83 g) and Topotecan hydrochloride form β had an HPLC purity of 99.7%.

### =Example 5 - Topotecan hydrochloride form α with different formaldehyde:dimethylamine ratios without alcohol

10-Hydroxy camptothecin (1.00 g) was dispersed in glacial acetic acid (20 ml), 40% aqueous formaldehyde (0.82 ml) and 40% aqueous dimethylamine (3.45 ml). The reaction temperature of the mixture was adjusted at 45°C and left under stirring until completion of the reaction (HPLC analysis), which required 2 hours; during this time the reaction mixture turned to a solution. After cooling to 10°C, 37% aqueous hydrochloric acid (3.40 ml) was dropped into the solution in about 5 minutes, without exceeding 12°C. The solvent was distilled at 50°C under pressure lower than 50 mbar to obtain a concentrated solution (8 ml). This residue was heated adjusting the temperature at 50°C; when the temperature of the concentrated mass reached 45°C, 95% aqueous isopropanol (50 ml) was added drop-by-drop in 90 minutes, without exceeding 52°C. The resulting crystallized product was allowed to stand for 60 minutes at 50°C, cooled to 20°C and after 90 minutes it was filtered with suction. The wet product thereby obtained was washed with 95% aqueous isopropanol (25 ml) and drying under vacuum in a static dried (residual pressure lower than 50 mbar) to afford Topotecan hydrochloride form α (1.10 g) with HPLC purity of al 99.8%.

## Claims

1. Topotecan hydrochloride **(Ia)** 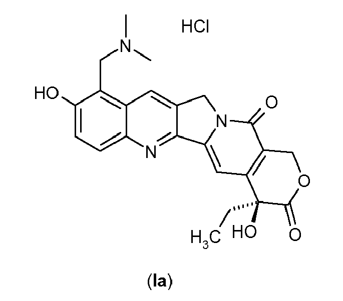 crystalline form α **characterized by** the XRPD spectrum X reported in the following table:
| **Angle (2-Theta)** | **Value d (Angstrom)** | **Intensity (%)** |
|---|---|---|
| 6.1 | 14.55 | 100.0 |
| 12.0 | 7.34 | 24.2 |
| 14.3 | 6.17 | 48.1 |
| 15.3 | 5.77 | 40.9 |
| 16.8 | 5.27 | 24.1 |
| 18.2 | 4.87 | 31.6 |
| 21.5 | 4.13 | 25.6 |
| 23.0 | 3.86 | 59.1 |

2. A process for the preparation of Topotecan hydrochloride form α as defined in claim 1 comprising the following steps:
a) reaction of 10-hydroxy-camptothecin **(II)** 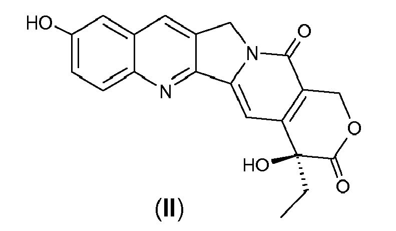 with an excess of aqueous formaldehyde and aqueous dimethylamine in acetic acid and a straight or branched C₂-C₄ alcohol;
b) addition of hydrochloric acid;
c) concentration of the reaction mixture;
d) crystallisation of Topotecan hydrochloride form α by addition of isopropanol or aqueous isopropanol;
e) recovery of the resulting Topotecan hydrochloride form α.

3. The process as claimed in claim 2 in which in step a) from 10 to 13 equivalents aqueous dimethylamine and from 4 to 18 equivalents aqueous formaldehyde are used.

4. The process as claimed in claim 2 or 3 in which the alcohol used in step a) is ethanol or isopropanol.

5. The process according to any one of claims 2 to 4 in which in step c) the reaction mixture is concentrated to a final volume of 8-10 volumes of solvent with respect to 10-hydroxy camptothecin.

6. Topotecan hydrochloride **(Ia)** 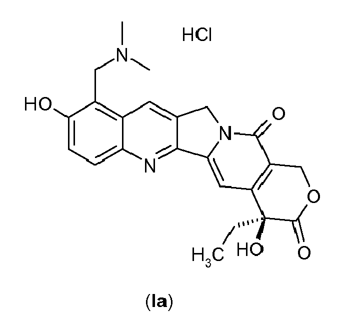 crystalline form β **characterized by** the XRPD spectrum reported in the following table:
| **Angle (2-Theta)** | **Value d (Angstrom)** | **Intensity (%)** |
|---|---|---|
| 5.3 | 16.59 | 100.0 |
| 11.7 | 7.56 | 21.7 |
| 13.1 | 6.76 | 11.6 |
| 15.5 | 5.73 | 8.8 |
| 16.0 | 5.55 | 38.4 |
| 16.6 | 5.35 | 5.4 |
| 17.2 | 5.14 | 6.0 |
| 20.0 | 4.44 | 13.9 |
| 25.4 | 3.50 | 10.4 |

7. A process for the preparation of Topotecan hydrochloride form β as defined in claim 6 comprising the following steps:
a) suspension of form α in aqueous isopropanol at a temperature ranging from 48 to 52°C for at least 60 minutes to obtain a crystalline suspension;
b) cooling of the crystalline suspension at a temperature ranging from 15 to 25°C;
c) recovery of Topotecan hydrochloride form β.

8. Pharmaceutical compositions containing Topotecan hydrochloride crystalline form α or β as defined in claims 1 and 6.
